# EUROPEAN PATENT APPLICATION

(11) **EP 1 314 738 A1**
(43) Date of publication of application: **28.05.2003**
(21) Application number: 01120261.1
(22) Date of filing: 23.08.2001
(51) Int. Cl.: C07K 14/025, C12N 15/37, A61K 39/12

(54) **Pseudovirons comprising virus capsids, histones and nucleic acids as therapeutic agents**

(71) Applicant: Virofem Diagnostika GmbH, 65174 Wiesbaden (DE)
(72) Inventor: Sapp, Martin Dr., 55278 Undenheim (DE)
(74) Representative: Grünecker, Kinkeldey, Stockmair & Schwanhäusser Anwaltssozietät

(57) **Abstract**

The invention relates to pseudovirions comprising virus capsids, histones, and nucleic acids, preferably DNA, in particular virus capsids and chromatin, as therapeutic agents and the use thereof in the treatment of diseases.

## Description

The invention relates to pseudovirions comprising virus capsids, histones, and nucleic acids, preferably DNA, in particular virus capsids and chromatin, as therapeutic agents and the use thereof in the treatment of diseases.

Human papillomaviruses (HPV) are widespread viruses causing benign or malignant lesions of the infected tissue. They exclusively replicate in differentiating cells of the skin and mucosa mainly inducing warts (or papillomas) and condylomata. Some lesions can progress to malignant intraepithelial lesions e.g. invasive cervical carcinoma, the second most frequent cancer of women worldwide. To date, more than one hundred types of HPV have been identified but only a few are strongly associated with malignant transformations.

Papillomaviruses contain a circular double-stranded DNA genome of approximately 8000 bp length, which is associated with histones to form a minichromosome. The icosahedral HPV capsid of T=7 symmetry is composed of the major and minor capsid proteins, L1 and L2. Capsids consist of 72 capsomeres each composed of five L1 molecules and probably twelve copies of L2 protein. Intercapsomeric disulfide bonds between two highly conserved cysteines covalently link L1 but not L2 protein.

It has been difficult to propagate HPV in cell culture at large scale because they depend on terminally differentiating keratinocytes for their replication. The expression of L1 and L2 in heterologous expression systems has allowed the high-yield production of virus-like particles (VLPs). These have been shown to be a good vaccine candidate and are amenable to genetic analyses. Recently several groups have succeeded in encapsidation of marker DNA. Two principally different approaches have been published.

One relies on dissociation and reassociation of partially purified VLPs in the presence of histone-free plasmid DNA thus producing pseudovirions harboring histone-free DNA.

The second approach depends on the expression of capsid proteins in cells carrying a marker plasmid at high copy number. In this case DNA in form of chromatin is packaged inside papillomavirus capsids. These in vivo-generated pseudovirions were used for the study of early events in papillomavirus infection using cultured cells and resulted in the identification of cell surface heparan sulfate as the candidate primary receptor.

Therefore, the object underlying the present invention is to provide a therapeutic agent based on pseudovirions.

The solution thereof is a pseudovirion comprising virus capsids, histones and nucleic acids as therapeutic agents.

The invention will be described in more detail below.

Figure 1 shows an analysis of cesium chloride gradients for L1 protein (curve), for infectivity (lined bars) and presence of pEGFPGFPNLS (grey bars). Asterisks indicate the fractions with a density of 1.33 g/cm³ (fraction 6) and 1.29 g/cm³⁽ (fractions 11 and 12), respectively.

Figure 2 shows an analysis of individual clones obtained after transformation of E. coli with DNA isolated from fraction 6 (1.33), fraction 12 (1.29) and fraction 14 (1.27) of the cesium chloride gradient shown in Fig. 1 (in vivo). Co means the analysis of the marker plasmid used for transfection of COS cells. All DNAs were purified and cut with EcoRI and BamHI prior to electrophoresis on 1 % agarose gels.

Figure 3A shows pseudovirions and VLPs generated after disassembly and reassembly in vitro which were analyzed in a non-reducing SDS-PAGE followed by L1-specific Western blot.

Figure 3B shows an aliquot of the same pseudovirions as shown in Fig. 3A which were treated with 0.025 % trypsin at 37°C for the indicated periods of time and subsequently subjected to an infectivity assay.

The virus capsids are preferably HPV virus capsids or VLPs derived from HPV. The VLPs may be obtained according to known procedures as described above. The VLPs preferably contain the major and minor capsid proteins of HPV, namely L1 and L2, in particular, comprising 72 capsomere, each composed of five L1 and optionally 12 L2 proteins.

The nucleic acids are preferably DNA.

The histones are preferably selected from the group consisting of histones H2a, H2b, H3, H4, H1 and mixtures thereof. In particular, the histones are selected from the group consisting of H2a, H2b, H3, H4, and mixtures thereof.

The histones and nucleic acids are preferably in the form of chromatin. In particular, the chromatin comprises DNA, and histones selected from the group consisting of H2a, H2b, H3, H4, H1 and mixtures thereof.

The pseudovirions used according to the present invention may be obtained in vivo, by transfecting appropriate cells with the DNA of interest, e.g. with a marker plasmid, and infecting these cells with recombinant vaccinia viruses for coexpression of capsid proteins. One possibility to generate pseudovirions in vivo is that COS cells transfected with the marker plasmid pGFPGFP-NLS are infected with recombinant vaccinia viruses for coexpression of HPV-33 L1 and L2. Pseudovirions were extracted from nuclei 40 hours postinfection by sonication in hypotonic buffer (10 mM Tris-HCl-1.5 mM MgCl₂-10 mM KCI, pH 7.5) supplemented with 0.5 % NP40. The lysate was clarified by centrifugation and cesium chloride was added to a density of 1.30 g/cm³. The samples were spun for 18 h at 55,000 rpm in a VTi65 rotor. Fractions were collected from the bottom and analyzed for the presence of L1 protein.

It was surprisingly found that chromatin-containing pseudovirions are fivefold more infectious than histone-free, i.e. naked DNA, containing pseudovirions.

The infectivity of pseudovirions generated in vivo and in vitro differs strongly. Pseudovirions generated *in vivo* harbor DNA in form of chromatin. In contrast, pseudovirions generated *in vitro* after disassembly and reassociation of purified VLPs in the presence of marker plasmid DNA contain histone-free naked DNA. Pseudovirions were generated in vitro using purified HPV-33 VLPs and pGFPGFP-NLS according to the procedure as described in J. Virol. 72:10298-10300.

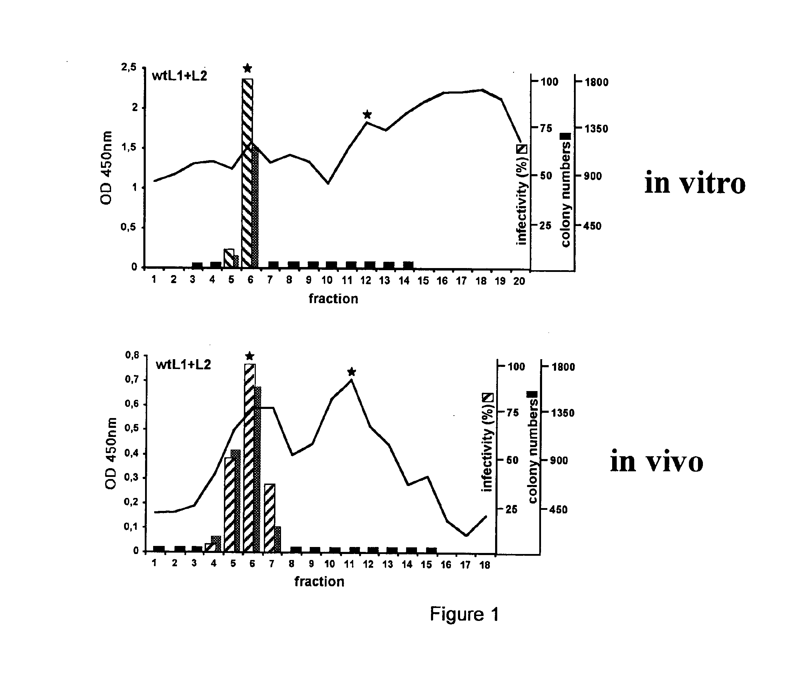

Fig. 1 shows the analysis of L1 protein across buoyant cesium chloride density gradients. Wt L1 protein was found to band in two broad peaks at densities of 1.29 and 1.33 g/cm³, respectively, when measured by L1-specific ELISA (indicated by asterisks). Pseudovirions obtained by either method contained L2 protein at the expected L1/L2 ratio of approximately 30:1.

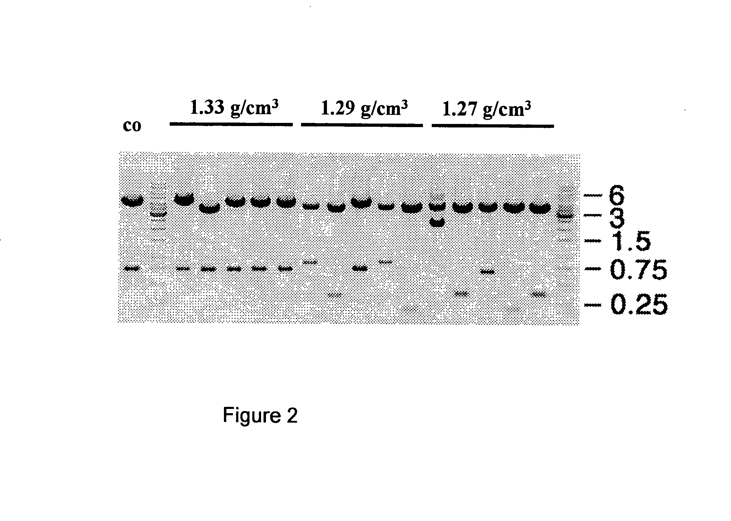

Next DNAse I-resistant marker plasmids were isolated from fractions across the gradients. To this end, aliquots of individual fractions were extensively dialysed for 6 h and subsequently treated with 250 µg/ml DNAse I for 1 h at 37°C to remove unpackaged DNA molecules. DNA was extracted by treatment with proteinase K followed by phenol/chloroform extraction and concentrated by ethanol precipitation. DNA was then used to transform E. coli. The number of colonies growing in the presence of kanamycin after transformation of E. coli served as direct measure for the amount of DNAse I-resistant i.e. encapsidated DNA molecules. Most fractions yielded a basic background of 20 to 40 colonies. Plasmid DNA analysis of individual clones from fractions with a background number of colonies revealed that most DNA molecules had undergone significant rearrangement and deletions since the isolated DNA differed from the control DNA in size (Fig. 2; 1.29 and 1.27 g/cm³). This indicates that the DNA was partially sensitive to DNAse I.

Fractions yielding kanamycin-resistant colonies significantly above background centered at a density of 1.33 g/cm³, where pseudovirions are expected to band as may be seen from Figure 1.

DNA analysis of randomly selected clones obtained from these fractions revealed that they mainly contained the input marker plasmid as can be seen in Figure 2 (1.33 g/cm³) indicating that these molecules were inaccessible for DNAse I.

Aliquots of the same fractions were also subjected to pseudoinfection assays. Infected cells were only observed when fractions containing DNA significantly above background were added to COS cells as may been seen in Figure 1. Infection was inhibited by preincubation of pseudovirions with homotypic VLP antiserum confirming that the DNA transfer is indeed capsid-mediated.

The numbers of encapsidated DNA molecules in pseudovirions obtained with either method were then related to the number of infectious units in the preparations. Estimation of the encapsidated DNA molecules revealed that four-to five-fold more DNA molecules were needed in case of pseudovirions harboring naked DNA to obtain the same number of infection events. The results of two independent experiments (exp. 1 and exp. 2) are given in Table 1.

**Table 1**

| Pseudovirions prepared | Number of colonies | Number of infections | Colonies/infections |
|---|---|---|---|
| in vitro exp. 1 | 6810 | 20 | 340 |
| exp. 2 | 8840 | 23 | 386 |
| in vivo exp. 1 | 14873 | 190 | 78 |
| exp. 2 | 18529 | 211 | 87 |

These data indicate that pseudovirions containing chromatin are four- to five-fold more infectious than pseudovirions harboring naked DNA.

Moreover, it is known from J. Virol. 75:7727-7731 that DNA encapsidation *in vivo* induces a conformational change in the papillomavirus capsid resulting in a 100 % degree of disulfide bonding of the L1 protein and a higher resistance to proteases.

To exclude that the differences in infectivity were due to differences in the structure of these two kinds of pseudovirions, the degree of disulfide cross-linking and the trypsin sensitivity of pseudovirions generated *in vitroK* was analyzed.

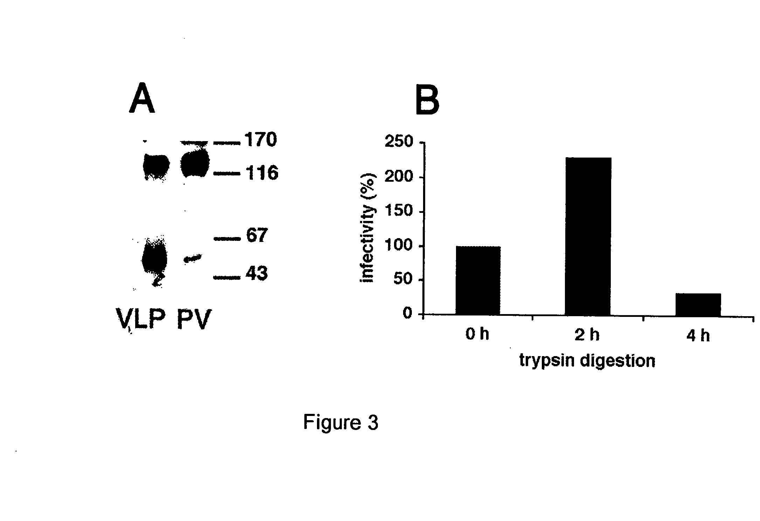

Fig. 3A shows that L1 protein, assembled to pseudovirions *in vitro,* was almost completely disulfide bonded suggesting that encapsidation of histone-free DNA induces a conformational change in the papillomavirus capsid, too. VLPs of the same gradient banding at a density of 1.29 g/cm³ displayed a 50 % degree of disulfide cross-linking. The DNA-induced structural change is confirmed by our observation that pseudovirions generated *in vitro* are also partially resistant to trypsin digestion (Fig. 3B).

Infectivity of pseudovirions even increased after treatment with trypsin for 2 h. Similar observations have been made for pseudovirions generated in COS cells. These results indicate that pseudovirions generated *in vivo* and *in vitro* have similar structural properties and that the increased infectivity of pseudovirions generated in COS cells is therefore due to chromatin.

The above data strongly indicate that encapsidation of chromatin renders pseudovirions more infectious than incorporation of histone-free DNA even though the structural properties seem to be very similar. Without being bound to a theory, this may be due to a more efficient nuclear translocation of the DNA once it is released from lysosomes into the cytoplasm. Alternatively, chromatin may be more resistant to nucleases than naked DNA and therefore may be more probable to reach its nuclear destination.

Therefore, pseudovirions comprising virus capsids, histones and DNA, in particular, histones and DNA in the form of chromatin, are very useful for the design of papillomavirus capsids as gene transfer vehicle.

Moreover, L2 protein seems to be useful for efficient gene-transfer into cell culture cells and should therefore preferably be included in a papillomavirus-based gene tranfer vector. The use of DNA in form of chromatin instead of histone-free plasmid DNA will add to the infectivity of papillomavirus-based pseudovirions.

Due to the superior effects of the pseudovirions used according to the present invention, these are useful as therapeutic agents, preferably selected from the group consisting of vaccines, anti-sense nucleic acids and gene transfer vectors.

The therapeutic agents of the present invention may be used in the treatment of diseases, in particular in the treatment of genetic disorders, infections, cancer and/or precancerous lesions. The therapeutic agents of the invention are in particular useful as prophylactic and/or therapeutic vaccines against infectious diseases, genetic disorders, cancers, precancerous lesions, autoimmune disorders and/or immunological disorders.

## Claims

1. Pseudovirions comprising virus capsids, histones and nucleic acids as therapeutic agents.

2. Pseudovirions according to claim 1, wherein the nucleic acids are DNA.

3. Pseudovirions according to claim 2, wherein the histones and DNA are in the form of chromatin.

4. Pseudovirions according to any one of the preceding claims, wherein the virus capsid is an HPV virus capsid or a VLP derived from HPV.

5. Pseudovirions according to claim 4, wherein the HPV virus capsid comprises the major and/or minor capsid proteins L1 and/or L2 and/or capsid proteins derived from L1 and/or L2.

6. Pseudovirions according to any one of the preceding claims, wherein the chromatin is composed of nucleic acid and histones H2a, H2b, H3, H4 and/or H1.

7. Pseudovirions according to claim 6, wherein the chromatin is composed of nucleic acid and histones H2a, H2b, H3, and/or H4.

8. Pseudovirions according to any one of the preceding claims, wherein the therapeutic agent is selected from the group consisting of vaccines, anti-sense nucleic acids, and gene transfer vectors.

9. Use of pseudovirions according to any one of the preceding claims in the preparation of a medicament for the treatment of diseases.

10. Use according to claim 9, wherein the diseases are selected from the group consisting of genetic disorders, infections, cancers, and precancerous lesions.

11. Use of pseudovirions according to any one of claims 1 to 8 in the preparation of a prophylactic and/or therapeutic vaccine against infectious diseases, genetic disorders, cancers, precancerous lesions, autoimmune disorders and immunological disorders.

12. Use of pseudovirions according to any one of claims 1 to 8 in the preparation of a gene transfer vehicle.
